# FASCICULE DE BREVET EUROPEEN

(11) **EP 0 950 662 B1**
(45) Date de publication et mention de la délivrance du brevet: **04.09.2002**
(21) Numéro de dépôt: 99400823.3
(22) Date de dépôt: 02.04.1999
(51) Int. Cl.: C07F 7/08, A61K 7/00

(54) **Composés fluoro-siliconés sous forme d'huile et leur utilisation en cosmétique**
Als Öl vorliegende Fluorsilicone und ihre Verwendung in der Kosmetik.
Fluorosilicon compounds as an oil, and their use in cosmetics

(30) Priorité: 15.04.1998 FR 9804682
(43) Date de publication de la demande: 20.10.1999
(73) Titulaire: L'OREAL, 75008 Paris (FR)
(72) Inventeur: Philippe, Michel, 91320 Wissous (FR); Henrion, Jean-Christophe, 93500 Pantin (FR)

(56) Documents cités:
- CHEMICAL ABSTRACTS, vol. 125, no. 12, 16 septembre 1996 Columbus, Ohio, US; abstract no. 146902, KONDO, HIROFUMI: "Lubricants and magnetic recording media using them" XP002086437 & JP 08 138233 A (SONY CORP, JAPAN)
- CHEMICAL ABSTRACTS, vol. 114, no. 2, 14 janvier 1991 Columbus, Ohio, US; abstract no. 8284, KAWAKAMI, SUSUMU ET AL: "Formation of fluorosiloxane coatings using the Langmuir-Blodgett technique" XP002086438 & JP 02 169067 A (NATOCO PAINT CO., LTD., JAPAN)

## Description

La présente invention a pour objet de nouveaux composés fluoro-siliconés se présentant sous la forme d'une huile du type non volatile et leur utilisation dans les domaines de la cosmétique et dans des compositions utiles en dermatologie, plus particulièrement dans celui des produits de maquillage ou de soins de la peau.

L'utilisation en cosmétique ou dans des compositions utiles en dermatologie de nouvelles huiles permettant d'améliorer les propriétés des compositions cosmétiques ou dermatologiques est particulièrement recherchée lorsque l'on souhaite notamment en améliorer la consistance et leur facilité d'application.

Par ailleurs, les huiles doivent posséder en elles-mêmes de bonnes propriétés permettant de conférer à la peau plus de souplesse et un toucher agréable.

De très nombreuses huiles ont déjà été proposées et sont couramment utilisées en cosmétique pour la réalisation de divers produits de maquillage.

Parmi ces huiles, deux grandes classes ont été développées ces dernières années et largement employées en cosmétique. Il s'agit d'une part, des huiles de silicone et, d'autre part, des huiles fluorées.

Toutefois, certaines de ces huiles ont l'inconvénient majeur de ne pas être compatibles avec d'autres huiles ou corps gras employés en cosmétique ou dermatologie.

Ce problème de compatibilité revêt une importance primordiale lorsque l'on souhaite obtenir des compositions présentant une bonne homogénéité.

Après diverses recherches effectuées sur un grand nombre de composés, on a constaté qu'une classe particulière de composés fluoro-siliconés présentait une très grande compatibilité avec diverses huiles, notamment hydrocarbonées, perfluorées et/ou siliconées.

L'état de la technique relatif aux composés fluoro-siliconés est essentiellement constitué par la demande JP-A-06256756 qui décrit, dans une composition hydrofuge pour le traitement de surfaces de verre, de céramique et de métaux, l'utilisation de composés fluoro-siliconés répondant à la formule : 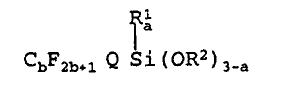 dans laquelle :
R₁ et R₂ sont des groupes monovalents hydrocarbonés en C₁-C₄,
Q est un groupe organique divalent en C₂-C₁₀,
a est 0 ou 1, et
b est 1 à 12.

La présente invention a donc pour objet, à titre de produits nouveaux, des composés fluoro-siliconés répondant à la formule générale (I) suivante : 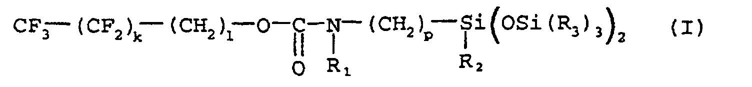 dans laquelle :
k est 1 à 17,
I est 1 à 18,
p est 1 à 6,
R₁ représente un atome d'hydrogène ou un radical alkyle en C₁-C₆,
R₂ représente un radical alkyle en C₁-C₆ ou le radical -OSi(R₃)₃, et
R₃ représente un radical alkyle en C₁-C₄.

Dans la formule ci-dessus, les radicaux alkyles en C1-C4 et C1-C6 sont de préférence choisis parmi les radicaux méthyle, éthyle, propyle, isopropyle, butyle, tertio-butyle et hexyle.

Parmi les composés répondant à la formule (I) ci-dessus, on peut notamment citer :
le N-(-2-F-octyl-éthyloxycarbonyl)-3-aminopropyl bis(triméthylsiloxy)méthylsilane,
le N-(-2-F-hexyl-éthyloxycarbonyl)-3-aminopropyl bis(triméthylsiloxy)méthylsilane,
le N-(-2-F-butyl-éthyloxycarbonyl)-3-aminopropyl bis(triméthylsiloxy)méthylsilane,
le N-(-2-F-octyl-éthyloxycarbonyl)-3-aminopropyl tris(triméthylsiloxy)silane,
le N-(-2-F-hexyl-éthyloxycarbonyl)-3-aminopropyl tris(triméthylsiloxy)silane, et
le N-(-2-F-butyl-éthyloxycarbonyl)-3-aminopropyl tris(triméthylsiloxy)silane.

La présente invention a également pour objet le procédé de préparation des composés de formule (I), celui-ci consistant à faire réagir, en milieu solvant organique, un chloroformiate de formule (Il) suivante : 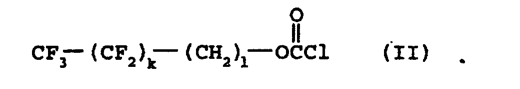 avec un aminosiloxane répondant à la formule (III) suivante : 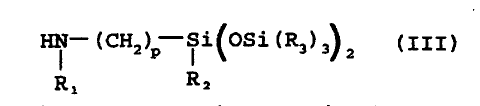 en présence d'une solution aqueuse basique. Avantageusement, le pH du milieu réactionnel est compris entre 8 et 12, de préférence entre 10 et 11.

La réaction de condensation est de préférence conduite à température ambiante et sous agitation. Après la fin de la réaction et addition d'eau, on acidifie, décante et sèche. Après évaporation du solvant, la distillation conduit à une huile incolore et sans odeur.

La présente invention a également pour objet une composition à application topique, notamment cosmétique ou dermatologique contenant en tant qu'huile, au moins un composé fluoro-siliconé de formule (I) telle que définie ci-dessus.

Dans la composition selon l'invention, le composé fluoro-siliconé est généralement présent en une proportion allant de 1 à 95 % en poids, de préférence 5 à 80 % et mieux de 5 à 60 %, par rapport au poids total de la composition.

Le composé huileux fluoro-siliconé selon l'invention présente, comme mentionné ci-dessus, une excellente compatibilité avec un grand nombre d'huiles, en particulier les huiles hydrocarbonées, les huiles perfluorées et/ou siliconées.

Parmi celles-ci on peut notamment citer l'huile de paraffine, l'huile de vaseline, l'isododécane, l'isohexadécane, les polyisobutènes, les polyisobutènes hydrogénés, les triglycérides d'acide caprique/caprylique, les cyclométhicones (en particulier en C₅), l'huile d'olive, l'octyldodécanol, la perfluorodécaline, le perfluorophénanthrène, les perfluorocycloalkyles et les perfluoropolyéthers.
Parmi les perfluorocycloalkyles, on peut notamment citer ceux de formule (IV) suivante : 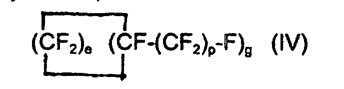 dans laquelle :
e est 4 ou 5,
g est 1 ou 2, et
p est 1, 2 ou 3 ;
sous réserve que lorsque g = 2, les groupements ne sont pas nécessairement en alpha l'un par rapport à l'autre.
Parmi les perfluoropolyéthers, on peut en particulier citer ceux répondant aux formules (V) et (VI) suivantes : 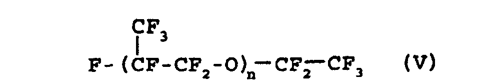 dans laquelle :
n est 7 à 30 ; et 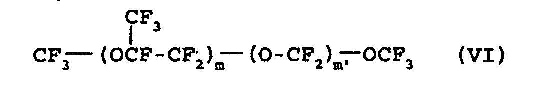
le rapport m/m' étant de 20 à 40.
Parmi ces perfluoropolyéthers de formules (V) et (VI), on peut tout particulièrement mentionner celui vendu sous la dénomination de "F_{LUORTRESS} LM36®" par la Société DUPONT, et ceux vendus sous la dénomination générale de "F_{OMBLIN}®" par la Société MONTEFLUOS, comme le "F_{OMBLIN} HCR®"
Les mélanges du composé huileux fluoro-siliconé selon l'invention et des huiles hydrocarbonées, perfluorées et/ou siliconées, éventuellement associées à d'autres corps gras, peuvent avantageusement constituer la phase grasse de diverses compositions cosmétiques qui peuvent être soit anhydres, soit aqueuses.
Lorsque les compositions sont du type anhydre, celles-ci peuvent se présenter sous la forme d'un gel anhydre, d'un stick tel que par exemple d'un rouge à lèvres ou d'un anti-cernes, ou sous la forme d'un compact anhydre tel que par exemple d'un fond de teint, d'un fard à paupières, d'un blush, d'un eye-liner ou d'un mascara, d'un maquillage du corps, ou encore de poudre libre. Dans ce cas, le composé fluoro-siliconé selon l'invention sert de liant, seul ou en mélange avec d'autres huiles.
Lorsque les compositions sont du type aqueux, celles-ci sont des dispersions sous forme d'une émulsion eau-dans-l'huile (E/H) ou huile-dans-l'eau (H/E), qui sont essentiellement constituées (i) d'une phase grasse, (ii) d'une phase aqueuse, et (iii) d'au moins un agent émulsionnant. Ces compositions peuvent être également sous forme d'une dispersion huile-dans-l'eau grâce à des vésicules lipidiques. Ces compositions ont alors l'aspect d'une crème ou d'un lait, blanc ou coloré (fond de teint fluide par exemple).
Bien entendu, les compositions selon l'invention peuvent contenir tout type d'ingrédient conventionnel utilisé dans les domaines concernés comme des conservateurs, des parfums, des charges, des colorants, des pigments, des gélifiants, des cires et des actifs cosmétiques ou dermatologiques.
On va maintenant donner à titre d'illustration, des exemples de préparation des composés fluoro-siliconés et de leur utilisation en cosmétique.

### EXEMPLE de PREPARATION

### Préparation du N-(-2-F-octyl-éthyloxycarbonyl)-3-aminopropyl bis(triméthylsiloxy)méthylsilane

Dans un réacteur de 1 litre surmonté de deux ampoules d'introduction, on introduit 50 g (95 mmol) de chloroformiate de 2-F-octyl-éthyle en solution dans 250 ml d'acétone. Après agitation du mélange à température ambiante, on ajoute goutte à goutte et simultanément en 1 heure, à l'aide de deux ampoules, les solutions A et B suivantes, de telle manière que le pH du mélange réactionnel soit compris entre 10 et 11.
Solution A : 26,56 g (95 mmol) de 3-aminopropyl bis(trimé-thylsiloxy) méthylsilane dans 100 ml d'acétone.
Solution B : 47,5 ml d'une solution aqueuse de soude 2N.
Après introduction des solutions A et B, on agite une heure supplémentaire à température ambiante, puis on ajoute au mélange réactionnel, 400 ml d'eau et 200 ml de dichloroéthane et on acidifie ensuite lentement à pH = 2 par addition d'HCl concentré.

Après décantation, on récupère la phase organique qui est lavée et séchée sur sulfate de sodium.

On évapore alors le solvant et t'huile brute obtenue est distillée sous pression réduite pour conduire à 27,1 g de N-(-2-F-octyl-éthyloxycarbonyl)-3-aminopropyl bis(triméthylsiloxy)méthylsila-ne sous forme d'une huile incolore et sans odeur.

### Analyses :

Eb : 135°-135,5°C / 0,40-,045 mBar
RMN ¹H (CDCl3) conforme à la structure
GC : mono-pic

| Analyse élémentaire : | | | | | | |
|---|---|---|---|---|---|---|
| % | C | H | O | N | F | Si |
| Calc. | 32,77 | 4,19 | 8,31 | 1,82 | 41,96 | 10,95 |
| Tr. | 32,70 | 4,15 | - | 1,77 | 42,60 | 11,26 |

### Préparation des autres composés fluoro-siliconés :

Selon le même mode opératoire que décrit ci-dessus, on a également obtenu sous forme d'une huile incolore et sans odeur, les différents composés énumérés aux pages 2 et 3.

### COMPOSITION COSMETIQUE

### Exemple 1 : Fond de teint sous forme de stick

On prépare selon l'invention un fond de teint sous forme d'un stick en procédant au mélange des ingrédients suivants :
- Dodécane 1,12 dioate de 2-F-octyl-éthyle 30 g
- N-(-2-F-octyl-éthyloxycarbonyl)-3-aminopmpyl bis (triméthylsiloxy) méthylsilane 60 g
- Pigments 10 g

Le composé fluoro-siliconé de ce fond de teint peut être avantageusement remplacé par l'un quelconque des autres composés énumérés aux pages 2 et 3.

### Exemple 2 : Crème de soin (émulsion eau-dans-huile)

- N-(-2-F-octyl-éthyloxycarbonyl)-3-aminopropyl bis (triméthylsiloxy) méthylsilane 10 g
- Tri-fluorométhyl C₁-C₄ alkyldiméthicone (Shin Etsu) 12 g
- Tri-perfluoro alkyl éthylcitrate (Zonyl TBC® de Dupont de Nemours) 8 g
- Céthyl diméthicone copolyol (Abil WE09® de Goldschmidt) 5 g
- Sel 0.7 g
- Eau qps 100 g

## Revendications

1. Composés fluoro-siliconés répondant à la formule générale suivante (I) : 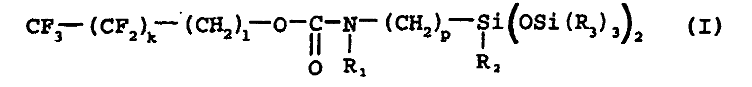 dans laquelle :
k est 1 à 17,
I est 1 à 18,
p est 1 à 6,
R₁ représente un atome d'hydrogène ou un radical alkyle en C₁-C₆,
R₂ représente un radical alkyle en C₁-C₆ ou le radical -OSi(R₃)₃, et
R₃ représente un radical alkyle en C₁-C₄.

2. Composés selon la revendication 1, **caractérisés par le fait que** les radicaux alkyles sont choisis dans le groupe constitué par les radicaux méthyle, éthyle, propyle, isopropyle, butyle, tertio-butyle et hexyle.

3. Composés selon l'une des revendications 1 et 2, **caractérisés par le fait qu'**ils sont choisis dans le groupe constitué par :
- le N-(-2-F-octyl-éthyloxycarbonyl)-3-aminopropyl bis(triméthylsiloxy)méthylsilane,
- le N-(-2-F-hexyl-éthyloxycarbonyl)-3-aminopropyl bis(triméthylsiloxy)méthylsilane,
- le N-(-2-F-butyl-éthyloxycarbonyl)-3-aminopropyl bis(triméthylsiloxy)méthylsilane,
- le N-(-2-F-octyl-éthyloxycarbonyl)-3-aminopropyl tris(triméthylsiloxy)silane,
- le N-(-2-F-hexyl-éthyloxycarbonyl)-3-aminopropyl tris(triméthylsiloxy)silane, et
- le N-(-2-F-butyl-éthyloxycarbonyl)-3-aminopropyl tris(triméthylsiloxy)silane.

4. Procédé de préparation des composés de formule (I) selon rune quelconque des revendications 1 à 3, **caractérisé par le fait qu'**il consiste à faire réagir, en milieu solvant organique, un chloroformiate de formule (Il) suivante 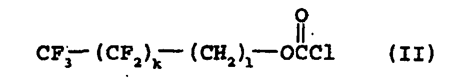 avec un aminosiloxane de formule (III) suivante : 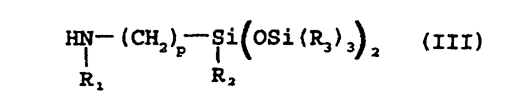 dans lesquelles k, I, p, R1, R2 et R3 sont tels que définis à la revendication 1, en présence d'une solution aqueuse basique.

5. Procédé selon la revendication 4, **caractérisé par le fait que** le pH du milieu réactionnel est compris entre 8 et 12.

6. Composition destinée à une application topique **caractérisée par le fait qu'**elle contient en tant qu'huile, au moins un composé fluoro-siliconé de formule (I) telle que définie aux revendications 1 à 3.

7. Composition selon la revendication 6, **caractérisée par le fait que** le composé fluoro-siliconé est présent en une proportion allant de 1 à 95 % en poids par rapport au poids total de la composition.

8. Composition selon l'une des revendications 6 et 7, **caractérisée par le fait que** le composé fluoro-siliconé est présent sous forme d'un mélange avec au moins une huile hydrocarbonée, perftuorée et/ou siliconée.

9. Composition selon l'une quelconque des revendications 6 à 8, **caractérisée par le fait qu'**elle est du type anhydre et se présente sous forme d'un stick, d'un compact, d'un mascara, d'un gel ou d'un eye-liner.

10. Composition selon l'une quelconque des revendications 6 à 8, **caractérisée par le fait qu'**elle est du type aqueux et se présente sous forme d'une émulsion eau-dans-l'huile ou huile-dans-l'eau, ou d'une dispersion huile-dans-l'eau grâce à des vésicules lipidiques.

11. Utilisation d'un composé fluoro-siliconé selon l'une des revendications 1 à 3, en tant qu'huile dans une composition cosmétique ou dermatologique.

## Patentansprüche

1. Fluorsilicone der folgenden allgemeinen Formel (I): 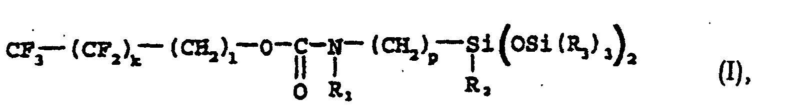 wobei:
k im Bereich von 1 bis 17 liegt,
l im Bereich von 1 bis 18 liegt,
p im Bereich von 1 bis 6 liegt,
R₁ Wasserstoff oder C₁₋₆-Alkyl bedeutet,
R₂ C₁₋₈-Alkyl oder eine Gruppe -OSi(R₃)₃ bedeutet, und
R₃ eine C₁₋₄-Alkylgruppe ist.

2. Verbindungen nach Anspruch 1, **dadurch gekennzeichnet, daß** die Alkylgruppen unter den Gruppen Methyl, Ethyl, Propyl, Isopropyl, Butyl, t-Butyl und Hexyl ausgewählt sind.

3. Verbindungen nach einem der Ansprüche 1 und 2, **dadurch gekennzeichnet, daß** sie ausgewählt sind unter:
N-(2-F-Octyl-ethyloxycarbonyl)-3-aminopropyl-bis(trimethylsiloxy)-methylsilan,
N-(2-F-Hexyl-ethyloxycarbonyl)-3-aminopropyl-bis(trimethylsiloxy)-methylsilan,
N-(2-F-Butyl-ethyloxycarbonyl)-3-aminopropyl-bis(trimethylsiloxy)-methylsilan,
N-(2-F-Octyl-ethyloxycarbonyl)-3-aminopropyl-tris(trimethylsiloxy)-silan,
N-(2-F-Hexyl-ethyloxycarbonyl)-3-aminopropyl-tris(trimethylsiloxy)-silan, und
N-(2-F-Butyl-ethyloxycarbonyl)-3-aminopropyl-tris(trimethylsiloxy)-silan.

4. Verfahren zur Herstellung von Verbindungen der Formel (I) nach einem der Ansprüche 1 bis 3, **dadurch gekennzeichnet, daß** es darin besteht, in einem organischen Lösungsmittelmedium ein Chlorformiat der folgenden Formel (II): 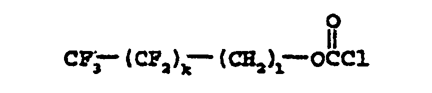 mit einem Aminosiloxan der folgenden Formel (III): 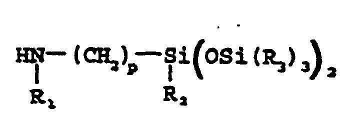 in Gegenwart einer wäßrigen basischen Lösung umzusetzen, wobei k, l, p, R₁, R₂ und R₃ die in Anspruch 1 angegebenen Bedeutungen aufweisen.

5. Verfahren nach Anspruch 4, **dadurch gekennzeichnet, daß** der pH-Wert des Reaktionsmediums im Bereich von 8 bis 12 liegt.

6. Zusammensetzung zur topischen Anwendung, **dadurch gekennzeichnet, daß** sie als Öl mindestens ein Fluorsilicon der Formel (I) nach einem der Ansprüche 1 bis 3 enthält.

7. Zusammensetzung nach Anspruch 6, **dadurch gekennzeichnet, daß** das Fluorsilicon in einem Mengenanteil von 1 bis 95 Gew.-%, bezogen auf das Gesamtgewicht der Zusammensetzung, vorliegt.

8. Zusammensetzung nach einem der Ansprüche 6 und 7, **dadurch gekennzeichnet, daß** das Fluorsilicon in Form eines Gemisches mit mindestens einem Kohlenwasserstofföl, perfluorierten Öl und/oder Siliconöl vorliegt.

9. Zusammensetzung nach einem der Ansprüche 6 bis 8, **dadurch gekennzeichnet, daß** sie wasserfrei ist und in Form eines Sticks, eines kompakten Produkts, eines Mascaras, eines Gels oder eines Eyeliners vorliegt.

10. Zusammensetzung nach einem der Ansprüche 6 bis 8, **dadurch gekennzeichnet, daß** sie wäßrig ist und als Wasser-in-Öl-Emulsion, Öl-in-Wasser-Emulsion oder Öl-in-Wasser-Dispersion vorliegt, die durch Lipidvesikel gebildet wird.

11. Verwendung eines Fluorsilicons nach einem der Ansprüche 1 bis 3 als Öl in kosmetischen oder dermatologischen Zusammensetzungen.

## Claims

1. Fluorosilicone compounds corresponding to the general formula (I) below: 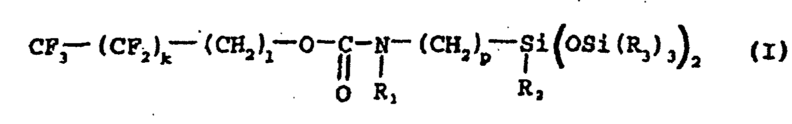 in which:
k is 1 to 17,
l is 1 to 18,
p is 1 to 6,
R₁ represents a hydrogen atom or a C₁-C₆ alkyl radical,
R₂ represents a C₁-C₈ alkyl radical or a radical -OSi(R₃)₃, and
R₃ represents a C₁-C₄ alkyl radical.

2. Compounds according to Claim 1, **characterized in that** the alkyl radicals are chosen from the group consisting of methyl, ethyl, propyl, isopropyl, butyl, tert-butyl and hexyl radicals.

3. Compounds according to either of Claims 1 and 2, **characterized in that** they are chosen from the group consisting of:
- N-(2-F-octylethyloxycarbonyl)-3-aminopropylbis(trimethylsiloxy)methylsilane,
- N-(2-F-hexylethyloxycarbonyl)-3-aminopropylbis(trimethylsiloxy)methylsilane,
- N-(2-F-butylethyloxycarbonyl)-3-aminopropylbis(trimethylsiloxy)methylsilane,
- N-(2-F-octylethyloxycarbonyl)-3-aminopropyltris(trimethylsiloxy)silane,
- N-(2-F-hexylethyloxycarbonyl)-3-aminopropyltris(trimethylsiloxy)silane, and
- N-(2-F-butylethyloxycarbonyl)-3-aminopropyltris(trimethylsiloxy)silane.

4. Process for preparing the compounds of formula (I) according to any one of Claims 1 to 3, **characterized in that** it consists in reacting, in organic solvent medium, a chloroformate of formula (II) below: 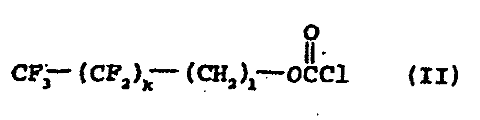 with an aminosiloxane of formula (III) below: 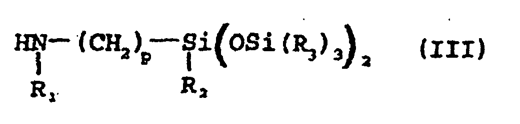 in which k, l, p, R₁, R₂ and R₃ are as defined in Claim 1,
in the presence of a basic aqueous solution.

5. Process according to Claim 4, **characterized in that** the pH of the reaction medium is between 8 and 12.

6. Composition intended for topical application, **characterized in that** it contains as oil at least one fluorosilicone compound of formula (I) as defined in Claims 1 to 3.

7. Composition according to Claim 6, **characterized in that** the fluorosilicone compound is present in a proportion ranging from 1% to 95% by weight relative to the total weight of the composition.

8. Composition according to either of Claims 6 and 7, **characterized in that** the fluorosilicone compound is present in the form of a mixture with at least one hydrocarbon-based oil, perfluoro oil and/or silicone oil.

9. Composition according to any one of Claims 6 to 8, **characterized in that** it is of the anhydrous type and is in the form of a stick, a compact, a mascara, a gel or an eyeliner.

10. Composition according to any one of Claims 6 to 8, **characterized in that** it is of the aqueous type and is in the form of a water-in-oil or oil-in-water emulsion, or an oil-in-water dispersion by means of lipid vesicles.

11. Use of a fluorosilicone compound according to one of Claims 1 to 3, as an oil in a cosmetic or dermatological composition.
